# EUROPEAN PATENT APPLICATION

(11) **EP 4 636 073 A1**
(43) Date of publication of application: **22.10.2025**
(21) Application number: 23902863.2
(22) Date of filing: 29.11.2023
(51) Int. Cl.: C12N 1/20, C12R 1/01

(54) **PERIBACILLUS ARACENENSIS BBB004 STIMULATOR OF PLANT ADAPTIVE METABOLISM AGAINST WATER STRESS, ENHANCER OF PLANT NUTRITION AND POLYPHENOL CONTENT**

(30) Priority: 15.12.2022 ES 202231072
(71) Applicant: Biobab R&D, S.L., 35260 Agüimes (Las Palmas) (ES)
(72) Inventor: Gutierrez Albanchez, Enrique, 28925 Alcorcón (Madrid) (ES); Horche Trueba, Ignacio, 28925 Alcorcón (Madrid) (ES); Ramos Solano, Beatriz, 28224 Pozuelo de Alarcon (ES); Gutierrez Mañero, Francisco Javier, 28691 Villanueva de la Cañada (Madrid) (ES); García-Villaraco Velascow, Ana, 28007 Madrid (ES); Lucas Garcia, José Antonio, 28760 Tres Cantos (Madrid) (ES)
(86) International application number: PCT/ES2023/070709
(87) International publication number: WO 2024/126879

(57) **Abstract**

Bacterial strain *Peribacillus aracenensis* (CECT 30655), microorganism from the group of Gram + bacteria, *Peribacillus* genus, stimulant of the adaptive metabolism of plants against water stress, improver of plant production and nutrition, and improver of polyphenol content. This strain, isolated from the rhizosphere of *Pinus pinaster,* on nutrient agar (PCA), has been characterized from a morphological, biochemical and genetic point of view by total sequencing of its genome, identifying it as a new species. It can be used to increase production in conditions of water stress, due to its ability to increase CO₂ fixation and transpiration, optimizing energy capture through photosynthesis and reducing oxidative stress; as a biofertilizer, due to its ability to improve nutrient absorption both in the presence and absence of water stress; and to improve the quality of fruits, due to its ability to increase antioxidant polyphenols in plant species of pharmacological and food interest.

## Description

The present invention refers to a strain of *Peribacillus aracenensis* (BBB004, internal laboratory code), as a new species of the *Peribacillus* genus, for use in the treatment of plants with the aim of improving production under conditions of water stress, increasing the CO₂ fixation and transpiration, optimizing energy capture through photosynthesis and reducing oxidative stress, with the consequent increase in growth and production. Also, it is capable of stimulating the absorption of various nutrients and increasing the polyphenol content, both under normal conditions and under the above-mentioned stress conditions, which is why it is also of interest as a biofertilizer for organic and conventional agriculture, and in order to increase the content of polyphenols in plant species of pharmacological and food interest, to improve the quality and durability of foods.

This strain has been filed for patent purposes in the Spanish Type Culture Collection (CECT, *Colección Española de Cultivos Tipo*), dated June 27, 2022, where it has been assigned the deposit number 30655. The CECT is based in the research building of the University of Valencia, located on the Burjassot campus (DP 46100 - Valencia, Spain).

### FIELD OF APPLICATION.

The invention belongs to the fields of agri-food biotechnology, specifically within the field of plant growth regulating bacteria, since the bacterial strain can be used as the bacterial strain can be used as a basis for the preparation of different types of products for increasing production under conditions of water stress, especially due to lack of water or osmotic stress. lack of water or osmotic stress, improving adaptation to sustained or punctual stress, increasing CO2 fixation, optimizing energy uptake through photosynthesis, and decreasing oxidative stress. These products will improve plant adaptation to different abiotic stress conditions, such as to different abiotic stress conditions, such as water deficit or excess soil salinity, improving production under conditions of low water supply.

It also falls within the field of organic biofertilizers, since the the strain can be used to improve plant nutrition in general, both in any bacterial formulation as well as with bacterial metabolites obtained under certain conditions.

### STATE OF THE ART.

Plants have several mechanisms to adapt to situations of water stress and salinity.

Photosynthesis is a physiological process that consists of transforming light energy into chemical energy according to the following equation: H₂O+CO₂ + E (Av) to produce C₆H₁₂O₆ + O₂, where water is absorbed by the roots and CO₂ by the leaves. It takes place in two major stages, i) the absorption of light energy and its transformation into chemical energy (ATP and NADPH), and ii) its subsequent use for CO₂ fixation, which is used to build the organic carbon skeletons. It is a process that requires photosynthetic pigments for energy uptake and generates high oxidative stress (free radicals - ROS). The plant has free radical scavenging systems to keep them in physiological limits, since, in turn, certain free radicals, such as H₂O₂, are physiological signals necessary for the proper functioning of the plant, for all this process is absolutely essential adequate water availability and good hydration of the plant. The uptake of CO₂ through stomata is vital for the proper functioning of photosynthesis, which generates a problem in contradiction with the requirements established for a good photosynthesis since it is the way out of water (transpiration), a process that is accelerated in adverse conditions of temperature and humidity.

In this way, the plant needs to be hydrated; the parameter that defines the hydric state of the plant is the hydric potential that in summary exposes the balance between water content (pressure potential) and solutes (osmotic potential). To do this, it is able to generate a negative osmotic potential with respect to the soil, so that water enters entrained by the solutes (ions) that are absorbed from the soil; these ions accumulate in the vacuoles, in turn forcing water into the vacuole, which maintains a water reservoir and keeps the plant turgid. The absorption of nutrients occurs through specific pumps and once absorbed they accumulate in vacuoles and/or are translocated to the aerial part thanks to the transpiration stream, and there they are incorporated into organic skeletons. Therefore, mineral nutrition requires water loss by transpiration.

Water stress can be due to a lack of water (drought) or to an excess of salts (salinity). When it is due to lack of water, the plant increases the synthesis of compatible solutes (organic molecules) to generate the negative osmotic potential and stay hydrated, keeping stomata open longer; if the drought situation is very pronounced, it closes them. When plants are in saline soils, they tend to accumulate Na+ ions, which, in high concentrations, are toxic to the plant. One of the adaptive mechanisms is to pump Na+ ions to the outside to prevent their accumulation. This response involves the generation of ROS, which activates the stress hormone response (activation of specific protein synthesis and increase of Ethylene (Et), Jasmonic (JA) and Abscisic Acid (ABA); ABA is responsible for stomata closure.

Thus, the plant must maintain a balance between the opening and closing of stomata to allow the exchange of CO2 and water, prioritizing stomata closure when water is limiting. In addition to stomata closure, the plant, as mentioned above, also has mechanisms to retain water, increasing the concentration of internal solutes (osmolytes, ions and other organic molecules), and antioxidant systems to ensure a good general state with efficient photosynthesis, keeping free radicals at physiological levels.

This brief description of the plant's mechanisms for adapting to water and salinity stresses highlights the many possible points of improvement to increase crop production; in particular, the use of PGPB (Plant Growth Promoting Bacteria) to improve the plant's ability to adapt to such adverse conditions.

There is a growing body of scientific evidence showing that beneficial bacteria are able to modify several plant targets simultaneously, i.e., they use more than one mechanism of action, which also varies according to the needs of the plant and is always aimed at improving plant adaptation (Ilagunamaran and Smith, 2017, doi: 10.3389/fpls.2017.01768). The mechanisms of action of plant growth-promoting bacteria can be summarized in two types: direct, when bacteria or their metabolites alter plant metabolism (hormonal activity, stimulation of adaptive mechanisms), and indirect, when they synthesize compounds that facilitate nutrient uptake or mobilization or prevent the growth of pathogenic microorganisms on the plant, without altering plant metabolism; this is not an exhaustive list. In the case of this patent, both are of interest. The plant has an adaptive metabolism, highly inducible, related to the adaptation of the plant to adverse situations that it has to face during its life and is susceptible to modification by PGPBs.

The genus *Bacillus* encompasses an extraordinarily diverse group of microorganisms currently comprised of 239 species/subspecies, ranging from pathogens to extremely useful strains in agriculture (Patel and Gupta, Int. J. Syst. Evol. Microbiol. 2020; 70:406-438, DOI 10.1099/ijsem.0.003775). Following the taxonomy of the Bergeys Manual, 2009 edition, this bacterium falls within the Domain Bacteria, Phylum *Firmicutes,* Class Bacilli, Order *Bacillales,* Family *Bacillaceae* (Logan NA, De Vos P. Genus Bacillus Cohn 1872. In: De Vos P, Garrity M, Jones D, Krieg R and Ludwig W (editors). Bergey's Manual of Systematic Bacteriology. New York: Springer; 2009. pp.21-128). Recently, from an exhaustive analysis of Bacillus genomes, the existence of six new genera has been described: *Peribacillus* gen. nov., *Cytobacillus* gen. nov., *Mesobacillus* gen. nov., *Neobacillus* gen. nov., *Metabacillus* gen. nov. and *Alkalihalobacillus* gen. nov. Nov, which allow a more appropriate classification of the species ascribed to the genus Bacillus previously (Patel and Gupta, 2020, doi 10.1099/ijsem.0.003775). The genus *Peribacillus* groups Gram-positive or Gram-variable, aerobic or facultatively aerobic, rod-shaped cells capable of forming endospores under environmental or nutritional stress conditions; most have been isolated from soil or animal intestines. The type strain is *Peribacillus simplex.*

Since Patel and Gupta described the genus *Peribacillus* in 2020, 8 new species have been described within this genus, *P. simplex, P. muralis, P. butanilovorans, P. louselieroae, P. gossypii, P. asahii, P. psychrosaccharolyticus and P. castrensis.* The latter (Rodriguez et al. 2022, doi: 10.3389/fpls.2022.896728), with beneficial effects for agriculture due to its ability to stimulate growth and its biocontrol capacity, i.e., involving mechanisms external to the plant.

In the case of the present patent application, new technologies have made it possible to identify a new species, *Peribacillus aracenensis* (BBB004, internal laboratory code), based on its unique genetic characteristics, which differentiate it from other *Peribacillus* strains when the genome of *P. aracenensis* BBB004 is compared with those of the currently available genomes of *Peribacillus* and *Bacillus.*

The parameters that allow the identification of a new species are some of the following: i) average nucleotide identity (ANI) and average amino acid identity (AAI), being the limit values to define a new species less than 95%; ii) DNA-DNA digital hybridization (dDDH), with values below 70%; iii) guanine-cytosine content (G+C), values above 1%; iv) tetranucleotide usage frequency correlation index (TETRA), with values below 0. 99; v) multilocus sequence analysis (MLSA), with values below 97%.

The genome of *P. aracenensis* strain BBB004 was first analyzed using tools available in EzBioCloud. First, the EzBioCloud identification service provides similarity-checked searches based on quality-controlled databases of 16S rRNA sequences. After that, ANI (average nucleotide identity), AAI (average amino acid identity), dDDH and TETRA analyses were performed. Type (Strain) Genome Server (TYGS) was used to analyze the whole genome.

First, the 16s gene of P. *aracenensis* BBB004 was analyzed in EzBiocloud, detecting the most similar species within the genus *Peribacillus: Brevibacterium frigotolerans* (reclassified as *P. frigotolerans* in 2020. https://doi.org/10.1099/ijsem.0.005389); *P. simplex, P. muralis* and *P. butanolivorans* with values less than 99.99% pairwise similarity. The highest similarity values were found with the first two (99.93 and 99.65%, respectively).

Comparing the genome of P. *aracenensis* BBB004 with the genomes of the 4 most similar species according to 16s, the following results were obtained: With P. simplex, the ANI and AAI values were 93.54% and 94%, respectively, both below the 95% threshold, the DNA-DNA digital hybridization (dDDH) values were 60.6%, below the 70% threshold, and the guanine-cytosine (G+C) content was 0.12%, below the 1% threshold. The tetranucleotide usage frequency correlation index (TETRA) values was 0.99822, below the threshold value 0.99. With *P. Frigoritolerans,* the following values are obtained: ANI and AAI 93.96 and 93.26%, respectively, both below 95%; DNADNA digital hybridization (dDDH) 59.2%, below 70%; and the difference in guanine-cytosine content (G+C) 0. 62, below 1%; finally, the tetranucleotide usage frequency correlation index (TETRA) values was 0.99704, equal to or greater than the reference 0.99%. Based on the results obtained from ANI, AAI and dDDH, we can state that *P. aracenensis* BBB004 is different from the two closest strains in the phylogenetic tree.

Therefore, based on the polyphasic approach, a new species has been described for which the name *Peribacillus aracenensis* sp. nov (CECT Deposit No. 30655) has been proposed. The physiological characteristics and genetic analysis of this strain allow it to be unequivocally identified, differentiating it from other species of the genus *Peribacillus.*

The *P. aracenensis* BBB004 strain is characterized by its beneficial effects on plants under water stress conditions, improving production: it improves CO2 fixation, keeping stomata open for a longer time, reflecting a stimulation of the metabolism involved in adaptation. It prevents the formation of free radicals and facilitates their elimination, improving plant adaptation to these conditions through a mechanism of homeostasis of redox metabolism. It also increases the water potential of the plant under these conditions, a clear indicator of the capacity of this strain to induce a water balance, resulting in a greater capacity of the plant to resist water stress conditions. Moreover, it is able to improve plant nutrition, enhancing plant growth and yield.

The fact that *Peribacillus* is a genus of bacteria discovered recently, in the year 2020, may be the explanation for the fact that there are no known published patents of the described species of this genus on any of the reported effects, so the present application constitutes, a priori, the first invention of a bacterial strain of the genus *Peribacillus* as PGPB.

### INVENTION.-

The object of the invention described hereof and which, in view of the state of the prior art, is understood to meet the conditions of novelty and inventive activity necessary to be worthy of the patent right, is the isolation and characterization of the bacterial strain *Peribacillus aracenensis* BBB004, with CECT deposit number 30655, which is a microorganism from the group of Gram + bacteria, *Peribacillus* genus, with a demonstrated ability to stimulate the metabolism of adaptation to water stress of plants, reducing oxidative stress, allowing greater fixation of atmospheric CO₂ and greater transpiration, optimizing energy capture and increasing plant growth and production in these conditions. It is also capable of enhancing the absorption of nutrients, in particular nitrogen, phosphorus, potassium, sulfur, calcium, magnesium, boron, manganese and zinc, improving mineral nutrition, and increasing the concentration of polyphenols, improving the antioxidant and preservative capacity of food, both under normal and stressful conditions.

The physiological characteristics and genetic analysis of this strain, with a genome sequenced in accordance with the WIPO ST.26 standard that is provided annexed to this descriptive report via XML file, allow it to be unequivocally identified, differentiating it from other species of the *Peribacillus* genus.

In a bacterial screening carried out in the rhizosphere of *Pinus pinaster,* a strain belonging to the *Peribacillus* genus was isolated, whose genetic analysis did not allow it to be placed in any of the known species of this genus.

Once isolated, a characterization of the repertoire of unique genes of *P. aracenensis* BBB004 was carried out. In the comparative analysis against the *Peribacillus* and *Bacillus* genomes, the BPGA program identified a total of 389 genes present exclusively in *P*. *aracenensis* BBB004, of which 324 genes were associated with a specific subsystem. Among the 133 unique genes of *P. aracenensis* BBB004, those involved in copper transport, and the synthesis and degradation of polyamines (which participate in processes such as the packaging of nucleic acids, modulation of membrane receptors and channels) stand out due to their abundance. ions, regulation of gene expression and cellular signaling) and nutrient absorption pumps. These 324 genes corresponding to SEQ ID NO: 1 to 324 of the sequence list.

After its characterization, various tests were carried out to reveal biochemical activities indicative of its potential capacity to mobilize nutrients and promote plant growth. These were auxin production, degradation of 1-aminocyclopropane-1-carboxylate, nitrogen mobilization, solubilization of phosphate, calcium, and production of siderophores and chitinases, having a positive effect for the production of siderophores. An API 50 CHB/E Medium was carried out, resulting positive for the degradation of carbon sources, L-Arabinose, Beta-Methyl-D-Xylose, Glucose, and L-Fucose.

Initially, experiments consisting of direct inoculation of the strain in a model plant (*Arabidopsis thaliana*) have been carried out, where it has induced a stimulation of the plant's adaptive metabolism that involves the signal transduction pathway mediated by ABA, JA and ET, activating the transcription of the PR1 and LOX2 genes. An increase in CO₂ fixation and an increase in perspiration were found in both cases.

Subsequently, another experiment was carried out in a greenhouse in tomato inducing water (osmotic) stress conditions for 6 weeks. An improvement in photosynthesis has been detected, decreasing the concentration of photosynthetic pigments and oxidative stress, increasing water potential.

Another experiment has been carried out in a greenhouse on tomato, inducing the plant with the bacteria and subjecting it to an osmotic stress shock. An improvement in photosynthesis has been detected, reducing the concentration of photosynthetic pigments and oxidative stress.

Another experiment was carried out on tomatoes, in the open air, maintaining strong water stress, limiting the supply of water (drought). This experiment revealed the ability of *P*. *aracenensis* BBB004 to increase CO₂ fixation and transpiration, decrease oxidative stress generated by photosynthesis, decreasing photosynthetic pigments and free radical production without compromising photosynthetic efficiency, and increasing non-enzymatic antioxidants. (polyphenols) and osmolytes (soluble sugars). When the water supply is not limited, an earlier flowering and increased CO₂ fixation are detected. An increase in production was detected when water conditions were limiting and an improvement in nutrient absorption.

Another greenhouse experiment has been carried out on rice, inducing the plant with the bacteria and subjecting it to an osmotic stress shock. *P. aracenensis* BBB004 is capable of improving CO₂ fixation and transpiration, reducing the concentration of photosynthetic pigments and oxidative stress when there is no stress. When an osmotic stress shock is applied, the effect on perspiration and pigments is maintained, but oxidative stress increases, indicating the activation of protective systems.

Another experiment has been carried out in a greenhouse on rice, inducing the plant with the bacteria and subjecting it to a water stress shock (drought) and assessing the recovery capacity. *P. aracenensis* BBB004 is capable of improving CO₂ fixation and transpiration, reducing the concentration of photosynthetic pigments and oxidative stress, indicating the immediate activation of protection systems against lack of water, which does not imply the accumulation of osmolytes.

Another experiment was carried out on olive trees on one-year-old seedlings, under controlled conditions, in the open air, maintaining high water stress, limiting the supply of water (drought) and in saline soil (osmotic). This experiment revealed the ability of *P*. *aracenensis* BBB004 to reduce oxidative stress generated by photosynthesis, increasing non-enzymatic antioxidants (polyphenols) and osmolytes (proline and soluble sugars), modifying the expression of genes involved in the maintenance of ionic homeostasis..

On the other hand, elicitation experiments have been carried out with *P. aracenensis* BBB004 in olive trees, limiting the supply of water during the plant's production cycle in order to increase production in well-established intensive cultivation and in full production (drought).. In particular, the application of the aforementioned bacterial strain in olive trees has been tested, limiting the supply of water during the summer months in order to increase production. It has been found that *P. aracenensis* BBB004 increases production, stimulating transpiration, decreases oxidative stress, decreasing photosynthetic pigments and increasing osmolytes (proline).

In another experiment with *P. aracenensis* BBB004, limiting the supply of water during the plant's production cycle in order to increase blueberry production, in intensive cultivation (drought). It has been found that *P. aracenensis* BBB004 increases blueberry production, stimulating CO₂ fixation, transpiration, reducing oxidative stress, photosynthetic pigments and increasing osmolytes (proline and soluble sugars) and phenols in leaves.

Finally, an experiment was carried out in olive trees, by applying *P. aracenensis* BBB004 on freshly pruned branches, finding increases in phenols of interest to human health, specifically oleuropein and its precursor secologanin in the leaf extracts, with a potential protective effect on the system. cardiovascular.

The aforementioned experiments on the use of *P. aracenensis* BBB004 as a production improver under conditions of water stress (osmotic and drought), an adaptation-to-stress and nutrition improver, are presented herein in the embodiment section.

The purpose of this invention and constituting the technical advantage thereof, is to have a bacteria which improves agricultural production in conditions of water stress (osmotic and/or drought), improving its nutrition, improving plant production. for its ability to optimize energy absorption, and increasing the efficiency of the photosynthetic process by improving carbon fixation, associated with a decrease in oxidative stress.

Consequently, this patent application claims the use of the *P. aracenensis* BBB004 strain, for its application in any type of plant species, forming part of any preparation, either individually or in combination with other organisms, with the purpose to improve plant nutrition, increasing agricultural production in conditions of water stress (osmotic and/or drought), improving the adaptation of plants to stress situations. Based on these effects on plants, *P*. *aracenensis* BBB004 improves plant nutrition and production (kg/ha) under water stress conditions.

### FIGURES.

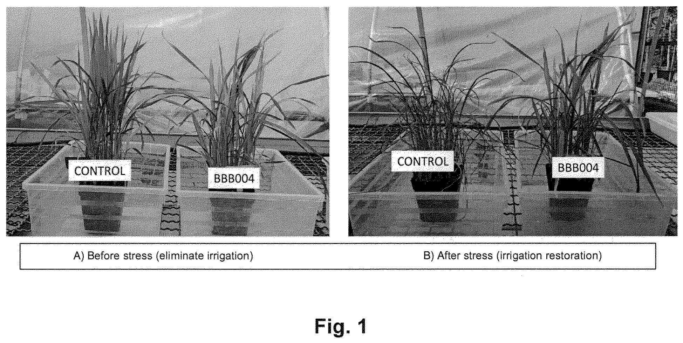

The images in **Figure 1****,** included at the end of this report, show the resulting appearance of rice plants in greenhouses after the experiments carried out on the performance of the strain, described in the following section.

### METHOD OF REALIZATION.

The strain of the genus *Peribacillus* described here was isolated by studying the rhizosphere of natural populations of *Pinus pinaster* in a transect of the Sierra de Aracena, in the province of Seville. This plant species (*Pinus pinaster*) was selected because it grows in nutrient-poor soils and has a low rainfall, distributed in strong occasional storms.

Rhizosphere sampling for isolation of the bacterial strain was carried out in natural populations of *Pinus pinaster* in October 2019. As a result of such sampling, *P. aracenensis* (BBB004, internal laboratory code) was found. The isolation of this strain was performed on nutrient agar (PCA).

In the laboratory, this microorganism is maintained with a high survival rate in 20% glycerol in nutrient broth (Pronadisa) at -80°C or in 15% glycerol in water at -20°C and is easily recovered in the culture medium used for isolation both in solid and liquid phase at 28°C.

### I. Morphological, biochemical and genetic characteristics of P. aracenensis BBB004.

For the characterization of the strains, different phenotypic characters were considered and are detailed in this report: (i) colony morphology, (ii) cell morphology, (iii) metabolic profile, (iv) comparative analysis of the genome of strain *P. aracenensis* BBB004 and the complete genomes available for the genus *Bacillus* and *Peribacillus.*
i) The morphology of the colonies at 24h of incubation at 28° in agar for standard methods (PCA) is specified in Table 1:

**TABLE 1**

| | BBB004 |
|---|---|
| Colony size | < 1 mmØ |
| Shape | circular |
| Border | Smooth |
| Transparency | No |
| Consistency | Creamy |
| Color | White |

Growing in liquid medium (LB LURIA BROTH) the color of the medium changes to yellow from the exponential phase of growth to the stationary phase of growth.
ii) The morphological characters of *P. aracenensis* BBB004 at 24h of incubation at 28° in agar for standard methods (PCA) correspond to a Gram-positive bacillus.
iii) Once isolated and characterized, with internal reference code BBB004, several tests were performed to demonstrate the PGPB potential of this bacterium. These were auxin production (Brick et al., 1991 Appl. Environ. Microbiol., 57(2), 535; Sergeeva et al., 2007, Plant Soil, http://doi.org/10.1007/s11104-007-9314-5), degradation of 1-aminocyclopropane-1-carboxylate (Glick et al., 1995. http://doi.org/10.1139/m95-070), nitrogen mobilization, phosphate solubilization (De Freitas et al., 1997. Biol. Fertil. Soils, 24(4), 358-364), calcium (Tamilselvi et al., 2016. Front. plant sci, 7, 1828), and siderophore production (Alexander and Zuberer, 1991. Biol.Fertil.Soils, 12(1), 39-45) and chitinases (Frändberg and Shunürer, 1998 Can.J. Microbiol. 44: 121-127).

This strain can grow in medium with 6% NaCl, and tolerates pH values between 5 and 7, not detecting growth at pH4 or above pH8. It can grow in a temperature range between 22°C and 40°C, losing viability above this value.

The metabolic profile (API 50 CHB/E Medium, https://us.vwr.com/store/product/29016242/api-50-chb-e-medium-biomerieux) of the strain indicates that it is capable of degrading carbon sources, L-Arabinose, β-Methyl-D-Xylose, Glucose, and L-Fucose.

iv) Comparison with the closest whole genome, *P. simplex* ANI and AAI values were 93.54% and 94%, respectively, both below the 95% threshold; DNADNA digital hybridization (dDDH) values, 60.6%, below the 70% threshold and guanine-cytosine (G+C) content 0.12%, above the threshold (1%). The tetranucleotide usage frequency correlation index (TETRA) values was 0.99822, above the reference threshold (0.99). Based on the ANI, AAI, dDDH and TETRA results, we can state that it meets the requirements of new species.

### II. Demonstrative experiments of the capacity of P. aracenensis BBB004 as an improver of plant nutrition and production under water stress conditions.-

**1°.** Demonstrative experiment of the capacity of the strain to activate the adaptive metabolism of the plant to abiotic stress conditions (salinity), in two phases.

First phase: No stress. Direct inoculation experiment of *P. aracenensis* BBB004 in model plant (*Arabidopsis thaliana*), compared with non-inoculated control. Experiment carried out in an experimental chamber under controlled conditions of light, humidity and temperature on a total of 42 plants and N = 3, with a randomized block model. A cell suspension of the strain was inoculated in pregerminated A. thaliana seedlings at 2 and 5 weeks after germination. At 6 weeks, photosynthetic parameters were measured, recording a significant increase in CO₂ fixation and transpiration; plants were harvested, and the expression of genes involved in the activation of adaptive metabolism was analyzed by RT-qPCR, via salicylic acid (SA) and ethylene/jasmonic acid (Et/JA) pathways. A slight increase in LOX expression was recorded, revealing an activation of the adaptive system (priming-pre challenge), which will manifest itself in a faster and more intense response when confronted with a stress factor.

Second phase: Salt stress. Similar experiment, subjecting *A.thaliana* plants stimulated with *P*. *aracenensis* BBB004 and non-inoculated control plants to a salt stress shock 5 weeks after germination: a significant increase in the expression of PR1, pdf1 and LOX was recorded compared to the non-inoculated control plant subjected to stress, revealing a simultaneous activation of the adaptation system via SA and Et/JA. There was a significant increase in CO₂ fixation and transpiration.

**2°.** Experiment of protection against sustained osmotic stress in tomato, in two phases. Elicitation experiment on Solanum lycopersicum var. Casillas plants by direct application of bacterial suspensions in the root, with the objective of demonstrating the improvement of plant growth and protection under sustained osmotic stress conditions.

First phase (no stress). A bacterial suspension of strain *P. aracenensis* BBB004 was inoculated into the root of tomato seedlings three times, the first time when they presented cotyledons and a pair of leaves, and the following two times every two weeks. At 6 weeks, photosynthetic parameters of fluorescence (F0, Fv/Fm, ϕPSII, NPQ), CO₂ fixation, and water potential were measured in both inoculated plants and non-inoculated controls, and harvested 5 days after the last application. The following parameters were analyzed: fresh and dry weight of plants, photosynthetic pigments, malondialdehyde (MDA) as a marker of cellular oxidative stress and H₂O₂, as a representative of reactive oxygen species (ROS); osmolytes (proline, soluble sugars and glycine betaine). In unstressed plants, an increase in dry weight (5%) of the aerial part was observed after inoculation, together with a decrease in photochemical fixed energy (ϕPSII) and a significant increase in energy dissipation (NPQ); a significant increase in CO₂ fixation, which supports growth enhancement. In addition, a decrease in photosynthetic pigments (10%), lower oxidative stress (MDA 13%) and lower osmolyte content (22% in proline and 4% in glycine betaine) are detected with respect to the non-inoculated control.

Second phase: Osmotic stress. Inoculated plants and non-inoculated controls were stressed with a 10% polyethylene glycol (PEG) solution applied during irrigation throughout the experiment to reproduce sustained osmotic stress conditions. An increase in fresh weight (27%) and dry weight (1%) of the aerial part, together with an improvement in the maximum photosynthetic potential (Fv/Fm), photochemical fixed energy (ϕPSII) and a decrease in energy dissipation (NPQ), indicating an optimization of energy absorption in photosynthesis, were observed in the inoculated plants under stress with respect to the non-inoculated control under stress. In addition, a decrease in photosynthetic pigments (12%) and lower oxidative stress (MDA, 13% and H₂O₂, 32%) were detected. An increase in water potential and a decrease in osmolyte content (25% proline and 7% betaine glycine) were also detected.

**3°.** Protection experiment against osmotic stress shock in tomato. Elicitation experiment in *Solanum lycopersicum* var. Casillas plants by direct application of bacterial suspensions in the root, under controlled conditions with the objective of demonstrating protection against an osmotic stress shock in inoculated plants and their non-inoculated controls. A bacterial suspension of the *P. aracenensis* BBB004 strain was inoculated in the root of tomato seedlings twice, the first time when they presented cotyledons and a couple of leaves, and the second, one week later; after 7 days, an irrigation with 10% PEG was applied to apply an osmotic shock, harvesting three days later. A similar response in the profile and intensity of biochemical modifications in photosynthesis, pigments, oxidative stress and osmolytes was observed in the inoculated plants subjected to stress. In addition to a significant increase in CO₂ fixation and transpiration with respect to the non-inoculated control subjected to osmotic stress.

**4°.** Experiment protection against drought stress, improving production and mineral nutrition in tomato. The experiment was conducted with *Solanum lycopersicum* var. Alcolea plants by direct application of bacterial suspensions in the root of plants, in open-air experimental plots, with limited water supply, maintaining the soil water potential at hydration limit conditions (between -300 and -400 hPa). The experiment was conducted from May to August 2022, with sustained very high temperatures (minimum 22°C, maximum 42°C). A bacterial suspension of *P. aracenensis* BBB004 was inoculated into the roots of tomato plants 6 times, the first time at transplanting, and the others every 15 days, with harvesting beginning two months after the first application; An intermediate sampling was carried out after the third application and the photosynthetic parameters of fluorescence (F0, Fv/Fm, ϕPSII, NPQ), CO₂ fixation, photosynthetic pigments, malondialdehyde (MDA) as a marker of cellular oxidative stress and H₂O₂, as a representative of reactive oxygen species (ROS); osmolytes (proline, soluble sugars and glycine betaine) and nutrient content were analyzed. A similar response in the profile and intensity of biochemical modifications was observed in treated plants compared to control plants without the strain in the drought stress situation, a significant increase in CO2 fixation (20%) and transpiration (26%), decrease in photosynthetic pigments (42% chlorophylls, 64% carotenes), lower oxidative stress (13% MDA) and modification of osmolytes, decrease in proline (26%) and increase in soluble sugars (15%). Nutrient content increased in plants treated with *P. aracenensis* BBB004 (Table 2). Yield increased by 91%, based on an increase in fruit number (40%) and fruit weight. Table 2 shows the nutrient content in tomato leaves after 3 applications under the stress conditions described, with irrigation limitation. Under normal conditions, an increase in nutrient uptake of nitrogen, magnesium and sulfur was observed.

**TABLE 2**

| | Nitrogen % | Phosphorus % | Potassium % | Sulfur % | Calcium % | Magnesium % | Boron mg/kg | Manganese mg/kg | Zinc mg/kg |
|---|---|---|---|---|---|---|---|---|---|
| Control R | 2.59 | 0.16 | 2.42 | 0.39 | 3.90 | 0.37 | 16.26 | 67.78 | 24.65 |
| BBB004 R | 2.63 | 0.25 | 2.77 | 0.53 | 100.35 | 0.39 | 42.24 | 82.17 | 31.40 |
| Increase over control | 1.56 | 50.92 | 14.64 | 35.63 | 35.63 | 6.66 | 159.77 | 21.23 | 27.38 |

**5°,** Protection experiment against osmotic stress in rice. An experiment was conducted on rice (*Oryza sativa* var Bahia), under controlled greenhouse conditions, on a total of 300 plants, with a randomized block model, including control and treated treatments with *P*. *aracenensis* BBB004, with and without PEG, as osmotic stress agent. A cell suspension of the strain was inoculated into pregerminated Oryza sativa seedlings at 4 and 5 weeks after germination. At 6 weeks, irrigation with PEG (25%) was applied in the appropriate treatments and after 3 days, photosynthesis (fluorescence and CO₂ fixation) and water potential measurements were taken and the plants were harvested.

In the non-stressed treated plants, a significant increase in CO2 fixation (28%), transpiration (8%) and water use efficiency (25%) was recorded with respect to the control plants without the strain; the maximum potential efficiency of the Fv/Fm system and ϕPSII increased, together with a decrease in photosynthetic pigments (32% chlorophylls and 21% carotenes) associated with similar levels of oxidative stress (MDA), and an increase in H₂O₂ (32%), a marker of priming that reveals an activation of the adaptive system (priming-pre challenge), which will manifest when faced with a stress factor. In addition, the osmolytes proline (11%), soluble sugars (4%) and glycine-betaine (5%) increased.

In treated plants subjected to an osmotic stress shock, a similar response to non-stressed plants was found in fluorescence and photosynthetic pigment parameters. However, CO₂ fixation decreased (50%) and transpiration increased (3%) in plants treated with *P*. *aracenensis* BBB004 with respect to their control, both under stress conditions. An increase in oxidative stress (30%) is detected together with an increase in H₂O₂ (70%); osmolytes decrease.

**6°.** Drought protection experiment (drought recovery capacity) in rice. In an experiment similar to experiment 5, the stress situation was applied 6 weeks after germination and after 2 inoculations, irrigation was suspended for 2 days and then irrigation was resumed and the same parameters were measured 24 hours later, when the plants had recovered (Figure 1). The behavior of photosynthesis followed the same pattern, with a decrease in photosynthetic pigments (49% chlorophylls and 40% carotenes), a decrease in oxidative stress (26%MDA) and H₂O₂ (60%) and a marked decrease in osmolytes, proline (50%), soluble sugars (37%) and glycine-betaine (7%). The significant increase in CO₂ fixation (74%), transpiration (37%) and water use efficiency (28%) in plants treated with *P. aracenensis* BBB004 with respect to the control, both subjected to the stress period, is very striking. Figure 1 shows the appearance of treated rice plants: A) before applying drought stress, in control plants without the strain and in those treated with BBB004; and, B) after stress (elimination of irrigation), when irrigation was restored, in control plants without the strain and treated with P. aracenensis BBB004.

**7°.** Experiment to improve adaptation to drought and salinity conditions in olive seedlings. An experiment was conducted on one-year-old Arbequina olive seedlings, in pots filled with saline soil (conductivity 6.07 dS/m), with irrigations every two weeks with saline water (8.20 dS/m), in the open air. A bacterial suspension of *P. aracenensis* BBB004 was inoculated in irrigations at root level. Applications were made every 15 days for 1 year. The number of plants in the trial was 6 per treatment. Sampling was performed after summer heat stress assessing fluorescence photosynthetic parameters (F0, Fv/Fm, ϕPSII, NPQ). Photosynthetic pigments, malondialdehyde (MDA) as a marker of cellular oxidative stress, osmolytes (proline, soluble sugars) and antioxidant polyphenols (flavonols and oleuropein) were analyzed. It was observed that treated plants compared to control plants without the strain in the initial stress situation, a decrease in photochemical fixed energy ϕPSII) and a significant increase in energy dissipation (NPQ). In addition, an increase in photosynthetic pigments (10%), higher osmolyte content (77% proline and 42% soluble sugars), more antioxidant polyphenols (flavonols +47%, oleuropein +25%) and lower oxidative stress (MDA-18%) were detected.

**8°.** Experiment to improve production by limiting open-air irrigation in olive trees. An experiment was carried out on irrigated super-intensive olive trees, reducing irrigation by 25% (eliminating one irrigation every 4 irrigations). The *P. aracenensis* BBB004 strain was tested in root application. Applications were made every 15 days from April to September (12 applications). The number of plants in the trial was 20 per treatment plus the control, with the two irrigation regimes (100% water and 75% water). Follow-up sampling (September) prior to harvest indicated that the *P. aracenensis* BBB004 strain applied at the root level caused an increase in yield (kg/ha) of 9% over the control plants without the strain with water reduction stress, with the same weight of olives treated with *P. aracenensis* BBB004 in the two irrigation regimes; the increase in fat yield was proportional (9%). In addition, photosynthetic parameters of CO₂ fixation, photosynthetic pigments, malondialdehyde (MDA) as a marker of cellular oxidative stress and osmolytes (proline, soluble sugars) of treated plants were analyzed with respect to the control with irrigation limitation, finding an increase in CO₂ fixation and transpiration (60%), decrease in photosynthetic pigments (40%), increase in water potential (13%), along with an increase in proline (87%) and decrease in soluble sugars (80%).

**9°.** Experiment to improve production with limited open-air irrigation in blueberry. Direct inoculation experiment of *P. aracenensis* BBB004 on blueberry (*Vaccinium corymbosum* var. Cupla) in production greenhouses. Experiment carried out under real field production conditions on a total of 21 plants per treatment (n=3, 7 plants per replicate) for each treatment (control and bacterium), with a randomized block experimental model. Cell suspensions of *P. aracenensis* strain BBB004 were applied at the root level during the 10-month production cycle, twice a month, from October to September; water was limited by 25% during the whole cycle. Total production was collected and a sampling time was determined, after summer stress, in September. Photosynthetic parameters of CO₂ fixation and water potential were determined. Photosynthetic pigments, malondialdehyde (MDA) as a marker of cellular oxidative stress, phenolic compounds as antioxidants, osmolytes (proline, soluble sugars) were analyzed. The treated plants, compared to the control plants without the strain in the water stress situation, showed an increase in fruit production (+33%), an improvement in CO₂ fixation (+8%) and in transpiration; in water potential (+21%), associated with an increase in proline (85%) and soluble sugars (120%); a decrease in photosynthetic pigments (40%) and an increase in antioxidant polyphenols (+272%), associated with a lower oxidative stress (-37% MDA).

**10°.** Experiment to improve the polyphenol content in olive pruning debris. A suspension of *P. aracenensis* BBB004 was applied to freshly pruned olive branches Arbequina var. They were allowed to dry and leaf extracts were prepared, for which leaves were pulverized, 80% ethanol was added, vortexed 45s and sonicated 30 min, followed by 5' centrifugation; the process was repeated 4 times; supernatants were pooled, brought to dryness, after resuspending in 5 mL of HPLC methanol, filtered (0.2µm nylon filters), and analyzed for polyphenol content by HPLC-MS. An increase in polyphenols with bioactive effect oleuropein (21%), secologanin (96%) and hydroxytyrosol (8%) was found from the treated pruning debris with respect to the controls without the strain.

### III. Demonstrative experiment of the ability of P. aracenensis BBB004 as a nutritional improver in Solanum lycopersicum in the absence of stress conditions.-

**1°.** Mineral nutrition improvement experiment in tomato. The experiment was conducted with *Solanum lycopersicum* Alcolea var. plants by direct application of bacterial suspensions in the root of plants, in open-air experimental plots in the absence of stress conditions, maintaining the soil water potential at optimal hydration conditions (between -100 and -200 hPa). The experiment was conducted from May to August 2022, with sustained very high temperatures (minimum 22°C, maximum 42°C). A bacterial suspension of *P. aracenensis* BBB004 was inoculated in the root of tomato plants 6 times, the first time at transplanting, and the others every 15 days. Nutrient measurements were made on leaves showing an improvement in nitrogen, sulfur, magnesium, and zinc nutrition of the treated plants with respect to the control without the strain.

**TABLE 3.**

| | Sulfur % | Nitrogen % | Magnesium % | Zinc mg/kg |
|---|---|---|---|---|
| Control R | 0.3035 | 2.2595 | 0.33265 | 29.94 |
| BBB004 R | 0.4585 | 2.8495 | 0.36625 | 31.7 |
| Increase over control | 51% | 26% | 10% | 6% |

### INDUSTRIAL APPLICATION. -

Given the above mentioned properties of *P. aracenensis* BBB004, it can be used to increase plant production under water stress conditions due to its ability to increase the photosynthetic process by improving carbon fixation, increasing transpiration, decreasing oxidative stress, and improving nutrient uptake. With respect to nutrient uptake, an increase was detected both in the presence and absence of water stress. It also improves metabolic performance, decreasing photosynthetic pigments and modifying the osmolyte profile, improving the plant's capacity to adapt to stress. It also increases the content of phenolic compounds, whose application would be to revalue plant remains as a source of active principles, in addition to improving the quality and durability of foodstuffs due to their antioxidant and preservative capacity.

## Claims

1. **Bacterial strain of the species *Peribacillus aracenensis,* with CECT deposit number 30655,** microorganism of the group of Gram + bacteria, *Peribacillus* genus, **characterized by** comprising the nucleotide sequences corresponding to SEQ ID NO: 1 to 324 of the sequence lists.

2. Bacterial strain of the species *Peribacillus aracenensis,* with CECT deposit number 30655, according to claim 1, **characterized by** its capacity to stimulate adaptive metabolism to water stress in plant species, increasing CO₂ fixation and transpiration, decreasing the concentration of photosynthetic pigments and oxidative stress, improving plant photosynthesis, plant growth and production.

3. Bacterial strain of the species *Peribacillus aracenensis,* with CECT deposit number 30655, according to claim 2, **characterized by** its ability to stimulate adaptive metabolism against water stress through simultaneous stimulation of signal transduction pathways mediated by SA and Et/JA.

4. Bacterial strain of the species *Peribacillus aracenensis,* with CECT deposit number 30655, according to claim 2, **characterized by** improving energy capture through photosynthesis and CO₂ fixation, increasing water use efficiency (WUE) and improving plant recovery after severe water stress, reducing oxidative stress.

5. Bacterial strain of the species *Peribacillus aracenensis,* with CECT deposit number 30655, according to claim 2, **characterized by** improving energy capture through photosynthesis and CO₂ fixation, increasing dry weight, both under sustained osmotic stress conditions and with osmotic shock, decreasing oxidative stress.

6. Bacterial strain of the species *Peribacillus aracenensis,* with CECT deposit number 30655, according to claim 1, **characterized by** its stimulating capacity for the absorption of nitrogen, phosphorus, potassium, sulfur, calcium, magnesium, boron, manganese and zinc in herbaceous and woody species, under conditions of water stress, improving mineral nutrition.

7. Bacterial strain of the species *Peribacillus aracenensis,* with CECT deposit number 30655, according to claim 1, **characterized by** its stimulating capacity for the absorption of nitrogen, sulfur, magnesium and zinc in herbaceous and woody species, in conditions of absence of water stress, improving mineral nutrition.

8. Bacterial strain of the species *Peribacillus aracenensis,* with CECT deposit number 30655, according to claim 1, **characterized by** its stimulating capacity of secondary metabolism in conditions of water stress, increasing the concentration of polyphenols in plant species, improving the quality and durability of foods due to its antioxidant and preservative capacity.

9. Use of the bacterial strain of the *Peribacillus aracenensis* species with CECT deposit number 30655, according to claims 1 and 2, for its application in any type of herbaceous and woody crop, in order to improve the adaptation of the plant to any water stress condition, whether natural, osmotic and/or drought, or due to lack of irrigation.

10. Use of the bacterial strain of the *Peribacillus aracenensis* species with CECT deposit number 30655, according to claims 1 and 6, for its application in any type of herbaceous and woody crop, with the aim of improving mineral nutrition in nitrogen, phosphorus, potassium, sulfur, calcium, magnesium, boron, manganese and zinc, against water stress, whether natural, osmotic and/or drought, or due to lack of irrigation.

11. Use of the bacterial strain of the *Peribacillus aracenensis* species with CECT deposit number 30655, according to claims 1 and 7, for its application in any type of herbaceous and woody crop, with the aim of improving mineral nutrition in nitrogen, sulfur, magnesium and zinc in conditions of absence of water stress.

12. Use of the bacterial strain of the *Peribacillus aracenensis* species with CECT deposit number 30655, according to claims 1 and 8, for its application in plant species of pharmacological and food interest, with the aim of improving the concentration of polyphenols, thus improving their antioxidant and preservative capacity.

13. Use of the bacterial strain of the *Peribacillus aracenensis* species with CECT deposit number 30655, according to claims 9 to 12, for its application in compositions, forming part of any bacterial preparation, either individually or in combination with other organisms, and by any available means that puts the bacteria in contact with the seed, the root or aerial system of the plants.
